# FASCICULE DE BREVET EUROPEEN

(11) **EP 1 879 654 B1**
(45) Date de publication et mention de la délivrance du brevet: **04.07.2012**
(21) Numéro de dépôt: 06744740.9
(22) Date de dépôt: 15.05.2006
(51) Int. Cl.: A61P 31/00, A61P 31/10, A61K 45/06, A61K 31/045, A61K 31/05, A61K 31/4196

(54) **COMBINAISON PHARMACEUTIQUE COMPRENANT UN AGENT ANTIFONGIQUE ET UN ACTIF QUI EST NOTAMMENT L'EUGÉNOL OU LE CARVACROL**
PHARMAZEUTISCHE KOMBINATION AUS EINEM FUNGIZID UND EINEM INSBESONDERE AUS EUGENOL UND CARVACROL AUSGEWÄHLTEN WIRKSTOFF
PHARMACEUTICAL COMBINATION COMPRISING AN ANTIFUNGAL AGENT AND AN ACTIVE SUBSTANCE SELECTED ESPECIALLY FROM EUGENOL AND CARVACROL

(30) Priorité: 13.05.2005 WO PCT/IB2005/001317
(43) Date de publication de la demande: 23.01.2008
(73) Titulaire: Advanced Scientific Developements, 20200 Casablanca (MA)
(72) Inventeur: REMMAL, Adnane, 30000 Fes (MA)
(74) Mandataire: Gallois, Valérie
(86) Numéro de dépôt international: PCT/IB2006/001329
(87) Numéro de publication internationale: WO 2006/120565

(56) Documents cités:
- WO-A-01/15680
- WO-A-96/40192
- WO-A-02/056879
- WO-A-03/049726
- WO-A-2004/070017
- WO-A-2004/089357
- US-A1- 2003 064 948
- US-A1- 2003 225 003
- DATABASE WPI Week 200382 Derwent Publications Ltd., London, GB; AN 2003-880974 XP002438179 & JP 2002 363070 A (YUTOKU YAKUHIN KOGYO KK) 18 décembre 2002 (2002-12-18)
- HUSSAIN, AZHAR A. ET AL: "Antifungal activity of Egyptian essential oils against some dermatophytes" AFRICAN JOURNAL OF MYCOLOGY AND BIOTECHNOLOGY (2003), 11(2), 1-20 CODEN: AJMBFV; ISSN: 1110-5879, 2003, XP008056733

## Description

L'invention concerne une composition pharmaceutique comprenant deux substances thérapeutiquement actives dont l'une exerce une action de potentialisation sur l'autre, ainsi que l'utilisation de cette composition.

Il est connu que l'efficacité des agents thérapeutiques dépend des doses utilisées, ce qui oblige, dans le cas des résistances partielles à augmenter les doses des agents thérapeutiques pour atteindre l'efficacité recherchée. Cette augmentation de la dose conduit à des problèmes d'apparition d'effets secondaires indésirables et de toxicité aiguë ou chronique, pouvant compliquer considérablement l'état des patients traités.

Cette résistance partielle peut devenir une résistance totale. Dans ce cas, l'augmentation des doses n'a plus aucun effet thérapeutique bénéfique, seuls les effets de toxicité sont observés. Le traitement consiste alors à changer l'agent thérapeutique.

Cette cascade peut se répéter et conduire à la situation la plus grave : la résistance totale à de multiples agents thérapeutiques (multi-drug resistance).

Ainsi, en particulier, les malades immunodéprimés, deviennent de plus en plus difficiles à traiter et leur espérance de vie en est réduite d'autant. De plus, leur confort de vie est largement affecté par l'administration à hautes doses d'agents thérapeutiques.

L'invention a pour but de pallier ces problèmes en proposant d'associer au moins deux substances thérapeutiquement actives, dont l'une potentialise l'activité de l'autre, ce qui permet non seulement d'abaisser les doses de chaque substance thérapeutiquement active mais également de traiter les patients atteints d'infections à germes résistants.

A cet effet, l'invention propose une composition pharmaceutique caractérisée en ce qu'elle comprend:
- au moins une première substance thérapeutiquement active choisie parmi l'eugénol et le le carvacrol, et
- au moins une seconde substance thérapeutiquement active qui est un antifongique et qui est choisi parmi le fluconazole, le voriconazole, la 5-fluorocytosine et la caspofungine.

La première substance thérapeutique peut être obtenue par synthèse chimique ou à partir d'une source végétale.

Une composition antifongique tout particulièrement préférée est une composition dans laquelle la première substance thérapeutiquement active est le carvacrol ou l'eugénol, et l'antifongique est le fluconazole.

Une autre composition antifongique tout particulièrement préférée est une composition dans laquelle la première substance thérapeutiquement active est le carvacrol ou l'eugénol, et l'antifongique est choisi parmi le voriconazole, la caspofungine, la 5-fluorocytosine, et leurs mélanges.

L'invention propose également une trousse (kit) caractérisée en ce qu'elle contient au moins un premier récipient contenant une première substance thérapeutiquement active choisie parmi l'eugénol le carvacrol, et au moins un second récipient contenant une seconde substance thérapeutiquement active qui est un antifongique comme défini plus haut.

L'invention propose enfin un traitement d'une infection due à un champignon caractérisée en ce qu'on administre à un patient atteint d'une infection due à un champignon, de manière simultanée ou séquentielle, au moins une première substance thérapeutiquement active choisie parmi l'eugénol et le carvacrol, et au moins une seconde substance thérapeutiquement active qui est un antifongique comme défini plus haut.

De préférence, on administre de manière simultanée ou séquentielle à un patient atteint d'une infection due à un champignon entre 10 et 200 mg/kg de poids du patient/jour de ladite première substance thérapeutiquement active, et entre 2 et 100 mg/kg de poids du patient/jour de ladite seconde substance thérapeutiquement active.

Ladite première substance thérapeutiquement active est choisie parmi le carvacrol et l'eugénol et ladite seconde substance thérapeutiquement active est choisie parmi le fluconazole, le voricinazole, la 5-fluorocytosine et la caspofungine, et leurs mélanges.

L'invention sera mieux comprise et d'autres buts et avantages de celle-ci apparaîtront plus clairement à la lecture de la description explicative qui suit et qui est faite en référence aux figures dans lesquelles :

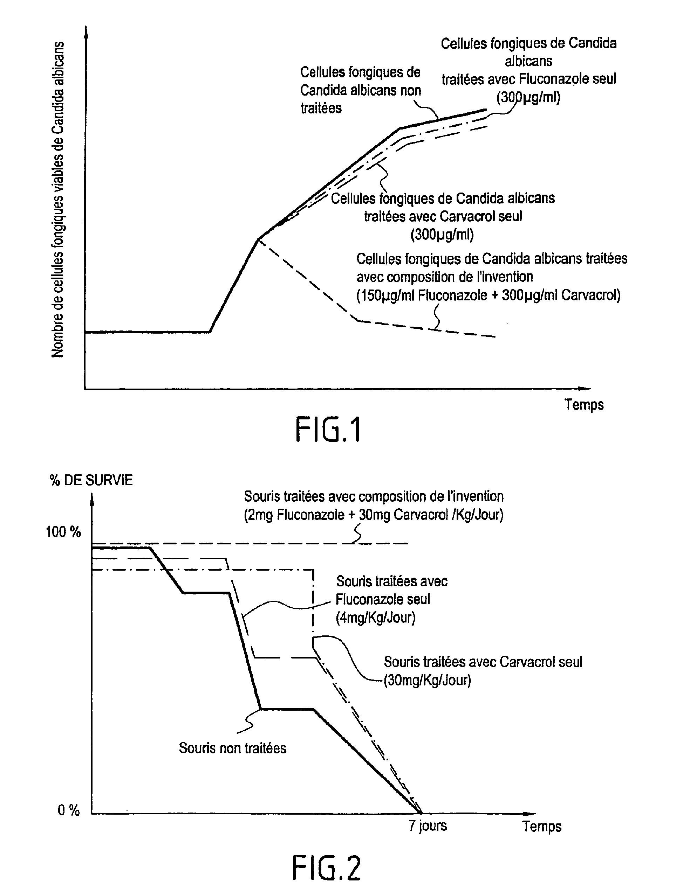

- la figure 1 montre les résultats des tests cinétiques de l'action fongicide, sur une culture de C.albicans, du fluconazole seul, du carvacrol seul par rapport à une composition pharmaceutique selon l'invention contenant du fluconazole et du carvacrol, et
- la figure 2 montre les résultats des tests *in vivo* sur un modèle de candidose systémique chez des souris expérimentalement infectées et soit non traitées, soit traitées avec du fluconazole seul, soit traitées avec du carvacrol seul, soit traitées avec une composition pharmaceutique selon l'invention contenant du fluconazole et du carvacrol.

La composition pharmaceutique de l'invention comprend en tant que première substance thérapeutiquement active l'eugénol ou le carvacrol, ainsi que leurs mélanges éventuels.

Ces composés ont des propriétés antifongiques bien connues en eux-mêmes et doivent être purs.

L'eugénol, le carvacrol se trouvent en proportions variées dans différents extraits de plantes aromatiques, c'est-à-dire qu'ils peuvent être purifiés à partir de ces plantes. Cependant, ils peuvent être tout simplement obtenus par synthèse chimique.

Or, les inventeurs ont maintenant découvert que ces composés ont un effet de potentialisation sur de nombreuses substances thérapeutiquement actives dont les agents antifongiques connus et déjà utilisés en tant que médicaments spécifiques de cette spécialité.

La seconde substance thérapeutiquement active comprise dans la composition pharmaceutique de l'invention est donc un antifongique, qui est déjà connu en tant que tel et déjà utilisé en tant que médicament spécifique de cette spécialité et dont l'activité est potentialisée.

Des exemples d'antifongiques connus et déjà utilisés en tant que médicaments spécifiques de cette spécialité qui peuvent être utilisés dans la composition pharmaceutique de l'invention, et dont l'effet sera potentialisé par la première substance pure thérapeutiquement active, appartiennent à trois familles : la famille des pyrimidines représentées par la 5-fluorocytosine, la famille des azolés représentés par le fluconazole, le voricinazole et la famille des échinocandines représentées par la caspofungine.

Ces composés peuvent être utilisés seuls, ou en combinaison l'un avec l'autre.

La composition pharmaceutique selon l'invention peut être formulée sous une forme adaptée pour une administration simultanée ou séquentielle desdites au moins première et seconde substances thérapeutiquement actives.

La forme galénique de la composition pharmaceutique de l'invention sera adaptée à son utilisation. Par exemple, elle pourra être utilisée sous la forme de solution, de suspension, de cachet ou autres. Les compositions pour administration parentérale sont généralement des solutions ou des suspensions stériles pharmaceutiquement acceptables qui peuvent éventuellement être préparées extemporanément au moment de l'emploi.

Pour la préparation de solutions ou de suspensions non aqueuses, on peut utiliser des huiles végétales naturelles telles que l'huile d'olive, l'huile de sésame ou l'huile de paraffine ou les esters organiques injectables tels que l'oléate d'éthyle. Les solutions stériles aqueuses peuvent être constituées d'une solution des substances thérapeutiquement actives dans l'eau. Les solutions aqueuses conviennent pour l'administration intraveineuse dans la mesure où le pH est convenablement ajusté et où l'isotonicité est réalisée, par exemple par ajout d'une quantité suffisante de chlorure de sodium ou de glucose.

En effet, étant donné la structure chimique des antifongiques, et d'autre part, vu la structure chimique du carvacrol et de l'eugénol, on pense, mais sans vouloir être lié par cette théorie, que le carvacrol et l'eugénol interagissent avec les antifongiques, pour former des complexes ayant une structure qui diffuse plus facilement dans les liquides physiologiques de l'organisme et qui diffuse plus facilement dans le cytoplasme des cellules infectées ciblées.

Mais, il a été démontré que lorsque les différents éléments de la composition pharmaceutique de l'invention sont mélangés en présence de détergents tels que le Tween ou le Triton ou de dissolvants tels que l'éthanol et le DMSO (diméthyl sulphoxide), les molécules actives de la première et de la seconde substance thérapeutiquement active s'associent avec les molécules de détergents et de dissolvants et ne forment pas de complexe de potentialisation.

Or on a découvert que le complexe de potentialisation se forme lorsqu'on utilise une suspension aqueuse d'agar, en tant que moyen de dispersion par viscosité.

Ainsi, la composition pharmaceutique de la présente invention serait de préférence préparée sans détergent et sans solvant. Par exemple, elle sera mise en suspension aqueuse rendue visqueuse par de l'agar à une concentration non gélifiante, par exemple de 1 à 5 grammes d'agar par litre de suspension.

La composition pharmaceutique de l'invention permet de traiter des infections localisées ou systémiques à germes résistants avec des doses plus faibles de chacune desdites première et seconde substances thérapeutiquement actives que les doses nécessaires au traitement des mêmes infections à germes sensibles, par l'une ou l'autre de ces mêmes dites première et seconde substances thérapeutiquement actives seules. En effet, la composition de l'invention permet d'utiliser des doses de ladite première substance thérapeutiquement active, lorsque en combinaison avec ladite seconde substance thérapeutiquement active, environ trois à dix fois inférieures à celles nécessaires lorsque ladite première substance thérapeutiquement active est utilisée seule et des doses de ladite seconde substance thérapeutiquement active, lorsque en combinaison avec ladite première substance thérapeutiquement active, de deux à dix fois inférieures à celles nécessaires lorsque ladite seconde substance thérapeutiquement active est utilisée seule.

Ceci a pour conséquence d'offrir un traitement qui présente les avantages suivants:
- efficacité contre les germes sensibles avec des doses très faibles,
- efficacité contre les germes résistants à un agent thérapeutique,
- efficacité contre les germes résistants à plusieurs agents thérapeutiques,
- lutte contre les phénomènes de récidive,
- lutte contre les phénomènes de sélection de germes résistants.

Dans tous ces cas, il y a une diminution remarquable des risques de toxicité et/ou d'apparition d'effets secondaires indésirables bien connus de l'homme du métier, grâce à la potentialisation qui permet l'administration de doses très faibles.

De plus, il en résulte une diminution du coût de production du traitement étant donné la faible quantité de principes actifs utilisés.

Les compositions pharmaceutiques de l'invention peuvent se présenter sous la forme de liposomes ou sous forme d'association avec des supports tels que les cyclodextrines ou les polyéthyléneglycols.

Les compositions pharmaceutiques de l'invention représentent un moyen simple et efficace pour lutter contre les problèmes liés aux agents microbiens en général qui sont essentiellement la résistance aux agents thérapeutiques et la toxicité de ceux-ci générée par l'utilisation de fortes doses.

En effet, l'éugénol et le carvacrol sont des molécules simples n'ayant jamais été décrites comme ayant une toxicité quelconque et leur ajout ayant un effet potentialisateur sur la seconde substance thérapeutiquement active permet d'utiliser des doses beaucoup plus faibles de cette seconde substance thérapeutiquement active.

Le procédé de traitement des patients atteints d'une infection fongique consistera donc, dans une première variante, à administrer à ces patients la dose déterminée par le médecin de la composition pharmaceutique de l'invention contenant les doses appropriées de ladite au moins une première substance thérapeutiquement active, combinées aux doses appropriées de ladite au moins une seconde substance thérapeutiquement active, c'est-à-dire l'antifongique approprié.

Dans une seconde variante, le procédé de traitement des patients atteints d'une infection fongique consistera à administrer à ces patients séquentiellement la dose déterminée par le médecin de ladite au moins une première substance thérapeutiquement active, puis la dose appropriée de ladite au moins une seconde substance thérapeutiquement active, c'est-à-dire l'antifongique approprié, ou l'inverse.

A cet effet, l'invention propose une trousse contenant, au moins un premier récipient contenant une desdites premières substances thérapeutiquement actives, et au moins un second récipient contenant une desdites secondes substances thérapeutiquement actives.

Cette trousse permettra au personnel soignant de préparer à la demande soit un mélange, des doses appropriées, de(s) la première(s) substance thérapeutique voulue(s) et de(s) l'antifongique(s) voulu(s), pour une administration simultanée, soit d'administrer séquentiellement et de façon séparée la dose appropriée de ladite au moins une première substance thérapeutiquement active, puis la dose appropriée de ladite au moins une seconde substance thérapeutiquement active, c'est-à-dire l'antifongique approprié, ou l'inverse. Cependant, on préférera utiliser un mélange pour utilisation simultanée pour permettre au complexe de potentialisation de se former et d'agir immédiatement dès l'administration au patient.

Pour mieux faire comprendre l'invention, on va maintenant décrire à titre d'exemples plusieurs modes de mise en oeuvre.

### EXEMPLE 1: Traitement de différentes souches de Candida albicans par le fluconazole potentialisé par le_carvacrol (Fluc P)

Tests in vitro : Détermination de la concentration minimale Fongicide (CMF) sur différentes souches de Candida Albicans

L'expérience a été menée avec plusieurs souches de Candida Albicans de sensibilités différentes isolées en milieu hospitalier au niveau buccal, vaginal et digestif. L'agent anti-fongique est le fluconazole qui est de la famille des dérivés azolés et qui fait partie des agents antifongiques les plus efficaces et les plus utilisés sur le marché. Une composition pharmaceutique antifongique a été fabriquée en mélangeant le fluconazole à différentes concentrations avec le carvacrol à une concentration infra inhibitrice de 0,3 gramme pour 1 I de solution ou d'excipient. Cette composition pharmaceutique de l'invention est notée FLUC-P pour fluconazole potentialisé. Dans chaque cas, l'activité antifongique a été testée soit avec du fluconazole seul, soit avec du carvacrol seul soit avec de la composition selon l'invention.

Le tableau 1 ci-après donne les résultats de tests statiques qui mesurent la concentration minimale fongicide (CMF en µg/ml).

**Tableau 1**

| *Candida albicans* en phase de croissance exponentielle | Fluconazole Seul | Composition selon l'invention | Carvacrol seul |
|---|---|---|---|
| | CMF (µg/ml) | CMF (µg/ml) | CMF (µg/ml) |
| Souche sensible | 0,5 | / | 1000 |
| Souche moyennement résistante | 150 | 10 | 1000 |
| Souche à forte résistance | >300 | 10 | 1000 |

Il apparaît du tableau 1 que la composition selon l'invention a une action fongicide remarquable sur ces souches de sensibilités variables, en comparaison au fluconazole seul ou au carvacrol seul.

En effet, on constate à la lecture du tableau 1 que la composition de l'invention a une action fongicide remarquable sur ces souches de sensibilité variable, comparé au fluconazole seul ou au carvacrol seul.

En effet, on constate, qu'en utilisant une concentration de 0,3 mg/ml de carvacrol, soit environ une concentration environ 3 fois inférieure à la CMF du carvacrol seul, la concentration en fluconazole permettant d'obtenir une efficacité fongicide est de 15 à 30 fois inférieure à la CMF du fluconazole seul.

### Tests cinétiques

Des tests cinétiques ont été aussi menés pour comparer les actions fongicides sur une souche de C. Albicans à forte résistance du fluconazole seul à une concentration de 300 µg/ml, du carvacrol seul à une concentration de 300 µg/ml et de la composition selon l'invention contenant 150 µg/ml de fluconazole et 300 µg/ml de carvacrol. Le nombre d'unités formant colonie, noté UFC, est mesuré dans le temps.

Les résultats sont montrés en figure 1. Il apparaît clairement de la figure 1 que seule la composition selon l'invention a une action probante sur cette souche de forte résistance.

### Tests in vivo :

Des lots de 15 souris ont été expérimentalement infectées par injection intraveineuse par 10 000 000 de cellules (Unités Formant Colonie) de Candida Albicans moyennement résistantes au fluconazole.

Le premier lot est constitué de souris témoins infectées non traitées.

Le deuxième lot est constitué de souris infectées et traitées par gavage, 24 h après l'infection, par du fluconazole seul à raison de 4 mg/jour par Kg de poids des animaux.

Le troisième lot est constitué de souris infectées et traitées par gavage, 24 h après l'infection, par du carvacrol seul à raison de 30 mg/jour par Kg de poids des animaux.

Le quatrième lot est constitué de souris infectées et traitées par gavage, 24 h après l'infection, à raison de 2 mg de fluconazole/kg de poids et 30 mg de carvacrol /kg de poids de souris.

Le traitement est de 7 jours pour les animaux qui restent en vie. On mesure dans le temps le pourcentage de souris survivantes. Les résultats de ces tests sont présentés en figure 2.

La figure 2 montre que seules les souris traitées avec la composition pharmaceutique sont encore vivantes dix jours après l'infection. Toutes les autres meurent entre le deuxième et le septième jour après l'infection.

La recherche de *Candida albicans* dans les organes des animaux morts pendant l'expérience (souris non traitées et souris traitées par le fluconazole seul ou le carvacrol seul) montre une forte charge du germe au niveau des reins, des poumons et de la moelle osseuse.

Par contre les animaux traités avec la composition selon l'invention sacrifiés entre 1 et 10 jours après l'arrêt du traitement montrent une absence du germe au niveau des poumons et de la moelle osseuse.

Au niveau des reins, deux animaux seulement portaient encore une très faible charge de *Candida albicans* équivalente à 5 % de la charge obtenue chez les animaux du lot témoin. Les autres animaux traités avec la composition selon l'invention n'avaient plus de charge fongique au niveau des reins.

Là encore, il apparaît clairement que la potentialisation du fluconazole par le carvacrol, permet d'obtenir des résultats surprenants quant à la diminution de la concentration minimale fongicide et à la vitesse d'action *in vitro.*

Cette potentialisation est retrouvée *in vivo* sur un modèle d'infection systémique.

Or, l'infection systémique représente une des formes d'infections les plus sévères qui menacent le plus la vie des patients et les plus difficiles à traiter surtout en cas de récidive avec des germes de plus en plus résistants.

D'autres résultats montrent que la composition selon l'invention contenant du fluconazole et du carvacrol donne des effets thérapeutiques surprenants à des doses au moins deux fois plus faibles que les doses nécessaires pour traiter les infections expérimentales localisées (vaginale et buccale), chez le rat et la souris.

### EXEMPLE 2 : Traitement de différentes souches de Candida albicans par du voriconazole potentialisé par le carvacrol (Vorico-P)

L'expérience a été menée avec plusieurs souches de *Candida albicans* de sensibilités différentes isolées en milieu hospitalier. L'agent antifongique est le voriconazole qui est de la famille des azolés et qui fait partie des agents antifongiques les plus récemment mis sur le marché. Une composition pharmaceutique antifongique selon l'invention a été fabriquée en mélangeant le voriconazole à différentes concentrations avec le carvacrol à une concentration infra inhibitrice de 0,3 g pour un litre de solution ou d'excipient. Cette composition pharmaceutique de l'invention est notée Vorico-P, pour voriconazole potentialisé. Dans chaque cas, l'activité antifongique a été testée soit avec du voriconazole seul, soit avec du carvacrol seul, soit avec la composition selon invention.

Le tableau 2 ci-après donne les résultats de tests statiques qui mesurent la concentration minimale inhibitrice (CMI) et la concentration minimale fongicide (CMF) en µg/ml.

**Tableau 2**

| *Candida albicans* en phase de croissance exponentielle | Voriconazole seul | Vorico-P | Carvacrol seul |
|---|---|---|---|
| | CMI (µq/ml) | CMF (µg/ml) | CMF (µg/ml) |
| Souche sensible | < 0,05 | / | 1000 |
| Souche résistante | 10 | <1 | 1000 |

Il apparaît du tableau 2 que la composition selon l'invention a une action fongicide remarquable sur la souche résistante au voriconazole en comparaison au voriconazole seul ou au carvacrol seul.
En effet, on constate à la lecture du tableau 2 qu'en utilisant le carvacrol à 0,3 mg/ml soit à une concentration 3,3 fois plus faible que la CMF du carvacrol seul, la concentration en voriconazole permettant d'obtenir une efficacité fongicide sur les souches résistantes est au moins dix fois inférieure à la concentration de voriconazole seul capable d'exercer une action fongistatique.

Ainsi, on constate que la potentialisation du voriconazole par le carvacrol permet non seulement de réduire considérablement la dose de voriconazole mais aussi de transformer son action fongistatique en une action fongicide.

### EXEMPLE 3: Traitement de différentes souches de Candida albicans par de la caspofungine potentialisée par le carvacrol (Caspo-P)

L'expérience a été menée avec plusieurs souches de *Candida albicans* de sensibilité différente isolées en milieu hospitalier. L'agent antifongique est la caspofungine qui est de la famille des échinocandines et qui fait partie des agent antifongiques les plus récemment mis sur le marché. Une composition pharmaceutique antifongique selon l'invention a été fabriquée en mélangeant la caspofongine à différentes concentrations avec le carvacrol à une concentration infra inhibitrice de 0,3 g pour un litre de solution ou d'excipient. Cette composition pharmaceutique de l'invention est notée Caspo-P, pour caspofungine potentialisée. Dans chaque cas, l'activité antifongique a été testée soit avec de la caspofungine seule, soit avec du carvacrol seul, soit avec la composition selon l'invention.

Le tableau 3 ci-après donne les résultats de tests statiques qui mesurent la concentration minimale inhibitrice (CMI) et la concentration minimale fongicide (CMF) en µg/ml.

**Tableau 3**

| *Candida albicans* en phase de croissance exponentielle | caspofungine seule | Caspo-P | Carvacrol seul |
|---|---|---|---|
| | CMI (µg/ml) | CMF (µg/ml) | CMF (µg/ml) |
| Souche sensible | < 0,05 | / | 1000 |
| Souche résistante | 3 | < 0,5 | 1000 |

Il apparaît du tableau 3 que la composition selon l'invention a une action fongicide remarquable sur la souche résistante en comparaison à la caspofungine seule ou au carvacrol seul.

En effet, on constate à la lecture du tableau 3 qu'en utilisant le carvacrol à 0,3 mg/ml soit à une concentration 3,3 fois plus faible que la CMF du carvacrol seul, la concentration en caspofungine permettant d'obtenir une efficacité fongicide est au moins six fois inférieure à la concentration de caspofungine seule capable d'exercer une action fongistatique.

Ainsi, on constate que la potentialisation de la caspofungine par le carvacrol permet non seulement de réduire considérablement la dose de caspofungine mais aussi de transformer son action fongistatique en une action fongicide.

### EXEMPLE 4: Traitement de différentes souches de Candida albicans par de la 5-fluorocytosine potentialisée par l'eugénol, notée Fluoro-P

L'expérience a été menée avec plusieurs souches de *Candida albicans* de sensibilité différente isolées en milieu hospitalier. L'agent antifongique est la 5-fluorocytosine qui est de la famille des pyrimidines et qui fait partie des agents antifongiques les plus anciens. Une composition pharmaceutique antifongique selon l'invention a été fabriquée en mélangeant la 5-fluorocytosine à différentes concentrations avec l'eugénol à une concentration infra inhibitrice de 0,5 g pour un litre de solution ou d'excipient. Cette composition pharmaceutique de l'invention est notée Fluoro-P, pour 5-fluorocytosine potentialisée. Dans chaque cas, l'activité antifongique a été testée soit avec de la 5-fluorocytosine seule, soit avec de l'eugénol seul, soit avec la composition selon l'invention.

Le tableau 4 ci-après donne les résultats de tests statiques qui mesurent la concentration minimale inhibitrice (CMI) et la concentration minimale fongicide (CMF) en µg/ml.

**Tableau 4**

| *Candida albicans* en phase de croissance exponentielle | 5-fluorocytosine seule | Fluoro-P | Eugénol seul |
|---|---|---|---|
| | CMI (µg/ml) | CMF (µg/ml) | CMF (µg/ml) |
| Souche sensible | 2,5 | / | 2000 |
| Souche résistante | 25 | 5 | 2000 |

Il apparaît du tableau 4 que la composition selon l'invention a une action fongicide remarquable sur la souche résistante en comparaison à la 5-fluorocytosine seule ou à l'eugénol seul.

En effet, on constate à la lecture du tableau 4 qu'en utilisant l'eugénol à 0,5 mg/ml soit à une concentration quatre fois plus faible que la CMF de l'eugénol seul, la concentration en 5-fluorocytosine permettant d'obtenir une efficacité fongicide est cinq fois inférieure à la concentration de 5-fluorocytosine seule capable d'exercer une action fongistatique.

Ainsi, on constate que la potentialisation de la 5-fluorocytosine par l'eugénol permet non seulement de réduire considérablement la dose de 5-fluorocytosine mais aussi de transformer son action fongistatique en une action fongicide.

### EXEMPLE 5: Elargissement du spectre d'action du Fluconazole potentialisé par le carvacrol pour agir sur différentes souches d'Aspergillus niger

L'expérience a été menée avec plusieurs souches *d'Aspergillus niger* insensibles au fluconazole. L'agent antifongique est le fluconazole qui est de la famille des azolés et qui fait partie des agents antifongiques les plus utilisés. Le plus grand inconvénient du fluconazole est qu'il n'agit pas sur les infections dues à des champignons filamenteux. Les infections les plus courantes et les plus difficiles à traiter sont les aspergilloses dues au germes de la famille *Aspergillus sp.* Une composition pharmaceutique antifongique selon l'invention a été fabriquée en mélangeant le fluconazole à différentes concentrations avec le carvacrol à une concentration infra inhibitrice de 0,25 g pour un litre de solution ou d'excipient. Cette composition pharmaceutique de l'invention est notée Fluc-P, pour fluconazole potentialisé. Dans chaque cas, l'activité antifongique a été testée soit avec de le fluconazole seul, soit avec de le carvacrol seul, soit avec la composition selon l'invention.

Le tableau 5 ci-après donne les résultats de tests statiques qui mesurent la concentration minimale inhibitrice (CMI) et la concentration minimale fongicide (CMF) en µg/ml.

**Tableau 5**

| *Candida albicans* en phase de croissance exponentielle | Fluconazole seul | Fluc-P | Carvacrol seul |
|---|---|---|---|
| | CMI (µg/ml) | CMF (µg/ml) | CMF (µg/ml) |
| 12 Différentes souches *d'Aspergillus niger* | > 1000 | 150 | 500 |

II apparaît du tableau 5 que la composition selon l'invention a une action fongicide remarquable sur les souches *d'Aspergillus niger* en comparaison au fluconazole seul ou au carvacrol seul.

En effet, on constate à la lecture du tableau 5 qu'en utilisant le carvacrol à 0,25 mg/ml soit à une concentration deux fois plus faible que la CMF du carvacrol seul mélangé avec le fluconazole à 150 µg/ml, nous arrivons à obtenir une efficacité fongicide remarquable que le fluconazole seul est incapable d'exercer même à la concentration de 1000 µg/ml.

Ainsi, on constate que la potentialisation du fluconazole par le carvacrol permet non seulement de réduire considérablement la dose de fluconazole avec les espèces habituellement traitées au fluconazole, mais aussi d'élargir son spectre d'action aux champignons filamenteux du genre aspergillus normalement insensibles au fluconazole.

Le traitement d'une affection fongique consistera à administrer, à un patient atteint d'une affection fongique, de manière simultanée ou séquentielle la dose déterminée par le médecin d'au moins une première substance thérapeutiquement active choisie parmi l'eugénol le carvacrol, et la dose déterminée d'au moins une seconde substance thérapeutiquement active qui est un agent antifongique comme défini plus haut.

Généralement, on administrera au patient atteint d'une affection due à des champignons, de manière simultanée ou séquentielle entre 1 et 3 000 mg/kg de poids du patient/jour d'au moins une première substance thérapeutiquement active choisie parmi l'eugénol et le carvacrol, et entre 1 et 20 mg/kg de poids du patient/jour d'au moins une seconde substance thérapeutiquement active qui est un agent antifongique comme défini plus haut.

En effet, l'utilisation d'une quantité inférieure à 1 mg/kg de poids du patient/jour de ladite première substance thérapeutiquement active, en particulier du carvacrol, ne permet pas d'obtenir l'effet de potentialisation voulu.

En revanche, l'utilisation d'une quantité supérieure à 3 000 mg/kg de poids du patient/jour de ladite première substance thérapeutiquement active, en particulier du carvacrol, ne permet pas d'augmenter l'effet de potentialisation et rapproche des risques de toxicité.

De la même façon, l'utilisation d'une quantité inférieure à 1 mg/kg de poids du patient/jour de ladite seconde substance thérapeutiquement active qui est un agent antifongique, en particulier de fluconazole, ne permet pas d'obtenir l'effet thérapeutique voulu et l'utilisation d'une quantité supérieure à 20 mg/kg de poids du patient/jour, en particulier de fluconazole, ne permet pas d'améliorer l'effet thérapeutique et augmente le risque de toxicité.

Ainsi, de préférence, on administre à un patient atteint d'une affection due à des champignons, de manière simultanée ou séquentielle, 30 mg/kg de poids du patient/jour d'au moins une première substance thérapeutiquement active choisie parmi l'eugénol, et le carvacrol, et 2 mg/kg de poids du patient/jour d'au moins une seconde substance thérapeutiquement active qui est un agent antifongique comme défini plus haut.

Plus particulièrement, dans le cas d'une affection due à *Candida Albicans,* on administre au patient, de manière simultanée ou séquentielle :
- 30 mg/kg de poids du patient/jour de carvacrol, et
- 2 mg/kg de poids du patient/jour de fluconazole.

## Revendications

1. Composition pharmaceutique **caractérisée en ce qu'**elle comprend :
- au moins une première substance thérapeutiquement active choisie parmi l'eugénol et le carvacrol, et
- au moins une seconde substance thérapeutiquement active qui est un antifongique choisi parmi la 5- fluorocytosine, le fluconazole, le voriconazole et la caspofungine.

2. Composition selon la revendication 1, **caractérisée en ce que** l'antifongique est le fluconazole.

3. Composition selon la revendication 1 ou 2, **caractérisée en ce que** la première substance thérapeutiquement active est le carvacrol.

4. Composition selon la revendication 1 ou 2, **caractérisée en ce que** la première substance thérapeutiquement active est l'eugénol.

5. Composition selon l'une quelconque des revendications 1 à 4, **caractérisée en ce que** lesdites première et seconde substances thérapeutiquement actives sont mises en suspension dans une solution aqueuse d'agar.

6. Composition selon l'une quelconque des revendications 1 à 5, **caractérisée en ce qu'**elle ne contient pas de détergent ou de solvant.

7. Trousse **caractérisée en ce qu'**elle contient :
- au moins un premier récipient contenant une première substance thérapeutiquement active choisie parmi l'eugénol et le carvacrol, et
- au moins un second récipient contenant une seconde substance thérapeutiquement active qui est un antifongique choisi parmi la 5- fluorocytosine, le fluconazole, le voricinazole et la caspofungine.

8. Trousse selon la revendication 7, **caractérisée en ce que** l'antifongique est le fluconazole.

9. Trousse selon la revendication 7 ou 8, **caractérisée en ce que** la première substance thérapeutiquement active est le carvacrol.

10. Trousse selon la revendication 7 ou 8, **caractérisée en ce que** la première substance thérapeutiquement active est l'eugénol.

11. Composition selon l'une quelconque des revendications 1 à 6 ou trousse selon l'une quelconque des revendications 7 à 10 pour une utilisation dans le traitement d'une infection due à un champignon chez un patient.

12. Composition ou trousse selon la revendication 11, **caractérisée en ce que** les doses à administrer sont :
- entre 10 et 200 mg/kg de poids du patient/jour pour la première substance thérapeutiquement active, et
- entre 2 et 100 mg/kg de poids du patient/jour pour la seconde substance thérapeutiquement active de ladite composition.

## Claims

1. A pharmaceutical composition **characterized in that** it comprises:
- at least one first therapeutically active substance selected from the group consisting of eugenol and carvacrol, and
- at least one second therapeutically active substance which is an antifungal agent selected from the group consisting of 5-fluorocytosine, fluconazole, voriconazole and caspofungin.

2. Composition according to claim 1, **characterized in that** the antifungal agent is fluconazole.

3. Composition according to claim 1 or 2, **characterized in that** said first therapeutically active substance is carvacrol.

4. Composition according to claim 1 or 2, **characterized in that** said first therapeutically active substance is eugenol.

5. Composition according to any one of claims 1 to 4, **characterized in that** said first and second therapeutically active substances are suspended in an aqueous agar solution.

6. Composition according to any one of claims 1 to 5, **characterized in that** said composition does not include any detergent or solvent.

7. Kit **characterized in that** it comprises:
- at least one first container containing a first therapeutically active substance selected from the group consisting of eugenol and carvacrol, and
- at least one second container containing a second therapeutically active substance which is an antifungal agent selected from the group consisting of 5-fluorocytosine, fluconazole, voriconazole and caspofungin.

8. Kit according to claim 7, **characterized in that** the antifungal agent is fluconazole.

9. Kit according to claim 7 or 8, **characterized in that** said first therapeutically active substance is carvacrol.

10. Kit according to claim 7 or 8, **characterized in that** said first therapeutically active substance is eugenol.

11. Composition according to any one of claims 1 to 6 or kit according to any one of claims 7 to 10 for use for the treatment of an infection caused by a fungus in a patient.

12. Composition or kit according to claim 11, **characterized in that** doses to be administrated are:
- between 10 and 200 mg/kg of body weight/day of the first therapeutically active substance, and
- between 2 and 100 mg/kg of body weight/day of the second therapeutically active substance of said composition.

## Patentansprüche

1. Pharmazeutische Zusammensetzung, **dadurch gekennzeichnet, dass** sie umfasst:
- wenigstens eine erste therapeutisch aktive Substanz, ausgewählt aus Eugenol und Carvacrol, und
- wenigstens eine zweite therapeutisch aktive Substanz, die ein Fungizid ist, ausgewählt aus 5-Fluorcytosin, Fluconazol, Voriconazol und Caspofungin.

2. Zusammensetzung gemäß Anspruch 1, **dadurch gekennzeichnet, dass** das Fungizid Fluconazol ist.

3. Zusammensetzung gemäß Anspruch 1 oder 2, **dadurch gekennzeichnet, dass** die erste therapeutisch aktive Substanz Carvacrol ist.

4. Zusammensetzung gemäß Anspruch 1 oder 2, **dadurch gekennzeichnet, dass** die erste therapeutisch aktive Substanz Eugenol ist.

5. Zusammensetzung gemäß einem der Ansprüche 1 bis 4, **dadurch gekennzeichnet, dass** besagte erste und zweite therapeutisch aktive Substanz in einer wässrigen Agarlösung suspendiert sind.

6. Zusammensetzung gemäß einem der Ansprüche 1 bis 5, **dadurch gekennzeichnet, dass** sie kein Detergens oder Lösemittel enthält.

7. Kit, **dadurch gekennzeichnet, dass** er enthält:
- wenigstens einen ersten Behälter, enthaltend eine erste therapeutisch aktive Substanz, ausgewählt aus Eugenol und Carvacrol, und
- wenigstens einen zweiten Behälter, enthaltend eine zweite therapeutisch aktive Substanz, die ein Fungizid ist, ausgewählt aus 5-Fluorcytosin, Fluconazol, Voriconazol und Caspofungin.

8. Kit gemäß Anspruch 7, **dadurch gekennzeichnet, dass** das Fungizid Fluconazol ist.

9. Kit gemäß Anspruch 7 oder 8, **dadurch gekennzeichnet, dass** die erste therapeutisch aktive Substanz Carvacrol ist.

10. Kit gemäß Anspruch 7 oder 8, **dadurch gekennzeichnet, dass** die erste therapeutisch aktive Substanz Eugenol ist.

11. Zusammensetzung gemäß einem der Ansprüche 1 bis 6 oder Kit gemäß einem der Ansprüche 7 bis 10 zur Verwendung in der Behandlung einer Pilzinfektion bei einem Patienten.

12. Zusammensetzung oder Kit gemäß Anspruch 11, **dadurch gekennzeichnet, dass** die zu verabreichenden Dosen:
- zwischen 10 und 200 mg/kg Körpergewicht des Patienten/Tag für die erste therapeutisch aktive Substanz, und
- zwischen 2 und 100 mg/kg Körpergewicht des Patienten/Tag für die zweite therapeutisch aktive Substanz besagter Zusammensetzung liegen.
